(19) Europäisches Patentamt / European Patent Office / Office européen des brevets

(11) **EP 1 404 291 B1**

(12) **EUROPÄISCHE PATENTSCHRIFT**

(45) Veröffentlichungstag und Bekanntmachung des Hinweises auf die Patenterteilung:
**24.09.2008 Patentblatt 2008/39**

(21) Anmeldenummer: **02743249.1**

(22) Anmeldetag: **29.06.2002**

(51) Int Cl.:
*A61K 8/35* (2006.01)          *A61K 8/86* (2006.01)
*A61K 8/92* (2006.01)          *A61K 9/107* (2006.01)
*A61K 9/48* (2006.01)          *A61K 31/122* (2006.01)
*A61Q 11/00* (2006.01)          *A61Q 19/00* (2006.01)

(86) Internationale Anmeldenummer:
**PCT/EP2002/007195**

(87) Internationale Veröffentlichungsnummer:
**WO 2003/007907 (30.01.2003 Gazette 2003/05)**

(54) **WASSERFREIES UBICHINON-KONZENTRAT**

WATER-FREE UBIQUINONE CONCENTRATE

CONCENTRE D'UBIQUINONE ANHYDRE

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **12.07.2001 DE 10133305**

(43) Veröffentlichungstag der Anmeldung:
**07.04.2004 Patentblatt 2004/15**

(73) Patentinhaber: **AQUANOVA AG**
**64295 Darmstadt (DE)**

(72) Erfinder: **BEHNAM, Dariush**
**64380 Rossdorf (DE)**

(74) Vertreter: **Blumbach - Zinngrebe**
**Patentanwälte**
**Saalbaustrasse 11**
**64283 Darmstadt (DE)**

(56) Entgegenhaltungen:
| | |
|---|---|
| **EP-A- 0 023 349** | **EP-A- 0 179 583** |
| **EP-A- 0 196 085** | **EP-A- 0 617 957** |
| **WO-A-01/52822** | **WO-A-98/21984** |
| **DE-A- 3 224 619** | **DE-A- 10 104 847** |
| **US-B1- 6 300 377** | |

- **DATABASE WPI Section Ch, Week 198031 Derwent Publications Ltd., London, GB; Class A96, AN 1980-54569C XP002217296 & JP 55 081813 A (NISSHIN FLOUR MILLING CO), 20. Juni 1980 (1980-06-20)**

Bemerkungen:
Die Akte enthält technische Angaben, die nach dem Eingang der Anmeldung eingereicht wurden und die nicht in dieser Patentschrift enthalten sind.

## Beschreibung

Substanzen wie Coenzym $Q_{10}$, Tocopherol, Isoflavone, Vitamin A usw. sind fettlöslich und im Unterschied zu wasserlöslichen Substanzen wie z.B. Ascorbinsäure nur zu geringen Teilen bioverfügbar und außerdem im klassischen Lebensmittelbereich aus technologischen Gründen nur mit großen Einschränkungen einsetzbar. Die nachfolgenden Erläuterungen sollen diesen Sachverhalt veranschaulichen.

Um die Vorteile des wasserlöslichen Coenzym $Q_{10}$ nachvollziehen zu können, sollte auf die Mechanismen der Fettverdauung und auf Emulsionen (Fett-Wasser-Gemische), die bei der Fettverdauung keine Vorteile bieten, näher eingegangen werden.

## 1.1 Fettverdauung

Die Ernährung hat den Sinn und Zweck, daß die lebenswichtigen Nährstoffe wie z.B. Vitamine, Mineralien, Spurenelemente vom Körper aufgenommen und verwertet werden. Die Aufnahme dieser Substanzen erfolgt durch die Schleimhautzellen im Dünndarm.

Auf den Zellen z.B. des Dünndarms liegt ein mikroskopisch feiner Wasserfilm, so daß die Zellen nur solche Substanzen unmittelbar aufnehmen können, die sich in diesem Wasserfilm lösen. Die Bioverfügbarkeit wasserlöslicher Substanzen wie z.B. Zucker, Salze und bestimmte Vitamine (z.B. Vitamin C) ist deshalb optimal.

Fettlösliche Substanzen hingegen - z.B. herkömmliches Coenzym $Q_{10}$ und die Vitamine E und A - können den Wasserfilm nicht durchdringen, sondern müssen im Dünndarm "vorbehandelt" werden. Dies geschieht auf dem Umweg der Micellenbildung mit Hilfe der Gallensekrete. Dieser "Umweg" ist der Grund dafür, daß die Aufnahme fettiger Substanzen nicht so einfach erfolgen kann wie bei wasserlöslichen Substanzen. Dieser Nachteil ergibt sich aus dem folgenden Sachverhalt:

1. Die Micellenbildung im Dünndarm erfolgt erst mit zeitlicher Verzögerung bzw. nach der Ausschüttung von Gallensekreten (Gallensaft) und Enzymen der Bauchspeicheldrüse.

2. Die Micellenbildung, die als Voraussetzung für die Fettverdauung gilt, erfaßt nur einen Teil der mit der Nahrung aufgenommenen Fette.

3. Während der vergleichsweise lang dauernden Bildung und "Einverleibung" der Micellen im Dünndarm laufen die übrigen Verdauungsvorgänge (Transport usw.) ununterbrochen weiter, so daß die gebildeten Micellen, die die Fettpartikel enthalten, zum größten Teil unverdaut ausgeschieden werden.

Der beschriebene Sachverhalt erklärt die sehr geringe Bioverfügbarkeit fettlöslicher Substanzen, die bei ca. 25 Prozent liegt. Für den Verbraucher bedeutet das, daß er einen großen Teil der fettlöslichen Substanzen, die er mit der Nahrung oder Nahrungsergänzungsmitteln wie z.B. fettlöslichen Coenzym $Q_{10}$-Kapseln zu sich nimmt, ungenutzt wieder ausscheidet. Darüber hinaus können manche Menschen aufgrund bestimmter Stoffwechselkrankheiten keine fettlöslichen Substanzen aufnehmen - es sei denn, daß diese in wasserlöslicher Form vorliegen (siehe beigefügtes Gutachten von Prof. Biesalski).

## 1.2 Emulsionen

Emulsionen sind trübe Fett-Wasser-Gemische, die für die Fettverdauung keinerlei Vorteile bieten. Sie zeigen die charakteristischen Eigenschaften von Fetten und Ölen bzw. fettlöslichen Substanzen (wie z.B. Coenzym $Q_{10}$). Diese Verbindungen sind oft leichter als Wasser und treiben deshalb in wäßrigen Lösungen wie auch im Magensaft an die Oberfläche. Gleichzeitig lagern sie sich aufgrund ihrer hydrophoben Wechselwirkungen aneinander und bilden durch diese Agglomeration oder Koagulation größere Gebilde.

Bei der großtechnischen Produktion von Emulsionen werden fettlösliche Verbindungen wie z.B. die Vitamine E und A mit Saccharoseacetatisobutyrat (SAIB, E 444) oder Glycerinester aus Wurzelharz (E 445) behandelt, um das spezifische Gewicht der fettlöslichen Verbindungen zu erhöhen. Auf diese Weise wird erreicht, daß die Fett- oder Ölpartikel nicht an die Oberfläche des wäßrigen Mediums steigen. Anschließend wird der Stabilisator Gummi arabicum (Arabisches Gummi, E 414) oder modifizierte Stärke (E 1450) hinzugegeben. Dadurch wird verhindert, daß die Fett- oder Ölpartikel zu größeren Gebilden (Tröpfchen) zusammenfließen. Im weiteren Verlauf werden die Fett- oder Ölpartikel durch Homogenisierung auf die Größe von ca. 1 μm zerkleinert.

Durch den beschriebenen Prozeß erhält man die Emulsion - ein trübes ÖI-WasserGemisch, das in der Verpackung zunächst stabil ist. Beim Verzehr wird es im Magen jedoch "zerstört", so das es für die Verdauung der emulgierten Fette oder Öle keinerlei Vorteile bietet Dieser Sachverhalt wird durch den folgenden Versuch deutlich:

Erwärmt man die (trübe) Emulsion auf Körpertemperatur und gibt Magensäure (Salzsäure) hinzu, so tritt sofort eine deutlich sichtbare Trennung in eine wäßrige und eine fettige Phase ein. Als Fazit bleibt festzuhalten, daß Emulsionen nichts mit Wasserlöslichkeit von Fetten zu tun haben.

## 1.3 Die revolutionäre Optimierung der Bioverfügbarkeit fettlöslicher Substanzen durch deren Umwandlung in ihre wasserlösliche Varianten

Aufnahme und Verwertung (Verdauung) von Fetten erfordern im Darm die Bildung von Micellen, so daß die Fette wie wasserlösliche Substanzen in die Zelle eindringen können. Wenn nun die Micellen bereits im Produkt in der Größe von ca. 50 nm vorliegen und darüber hinaus noch temperatur- und säurestabil sind, ist der körpereigene Vorgang der Micellenbildung überflüssig. In diesem Fall werden die Fette wie z.B. Coenzym $Q_{10}$ aus diesen Micellen wie wasserlösliche Substanzen vollständig vom Körper aufgenommen.

Nach diesem Prinzip ist u.a. das wasserlösliche Coenzym $Q_{10}$ entwickelt worden. Solubilate des wasserlöslichen Coenzyms $Q_{10}$ zeigen im Unterschied zu Emulsionen genau die gleichen Eigenschaften wie wasserlösliche Substanzen. Das Coenzym $Q_{10}$-Solubilat ist absolut klar und sogar bei 100°C und pH 1 noch absolut temperatur- und säurestabil. Auf diesen Sachverhalt ist die vierfach höhere und schnellere Bioverfügbarkeit zurückzuführen.

Die in großer Zahl bereits im Produkt befindlichen micellenartigen Einheiten, die die Fette (z.B. Coenzym $Q_{10}$) enthalten, sind stabil gegen die Temperatur- und Säureeinwirkungen im Magen. Sie gelangen unversehrt in den Dünndarm, lagern sich dort flächendeckend an die Schleimhautzellen an und können ungehindert und deshalb vierfach mehr und vierfach schneller von der Zelle aufgenommen werden, als dies bei der "normalen" Fettverdauung der Fall ist.

Für den Konsumenten bedeutet diese Tatsache einerseits einen wirtschaftlichen Vorteil, und andererseits die Gewißheit, daß das, was er erwirbt, rechtzeitig wirkt.

## 2 Die besondere Vorteile der zu patentierenden Erfindung

Die Bildung von Micellen ist für die Verdauung bzw. für die zelluläre Fettresorption absolut entscheidend und unabdingbare Voraussetzung. Sie erfolgt entweder mit Hilfe von Gallensekreten und Enzymen im Dünndarm oder gemäß der hier beschriebenen Erfindung bereits im Produkt. Die erfindungsgemäß im Produkt gebildeten Micellen müssen die folgenden Voraussetzungen erfüllen, um im Verdauungstrakt nicht als fett-, sondern als wasserlösliche Substanzen behandelt zu werden, so daß im Dünndarm keine besondere Micellenbildung mehr erforderlich ist:

1. Eine möglichst geringe Größe:

Je kleiner die Micellen im Produkt sind, desto geringer ist die Trübung im Trübungstest, wie aus der nachfolgenden Tabelle hevorgeht.

2. Magensäureresistenz

3. Langzeitstabilität im Produkt:

Die Langzeitstabilität im Produkt ist nur dann gegeben, wenn das Coenzym $Q_{10}$ nach langer Verweilzeit nicht auskristallisiert und bei 37°C transparent bleibt.

Das wasserfreie wasserlösliche Coenzym $Q_{10}$-Konzentrat bietet bei einem Einsatz in Kapseln - besonders in gelatinefreien Kapseln (VegaGel), den Verbrauchern die Vorteile von zwei grundlegenden Neuentwicklungen in einem einzigen Produkt.

Daneben bietet wasserlösliches Coenzym $Q_{10}$ die folgenden Möglichkeiten:

a) Der Kosmetikindustrie bietet sich die Möglichkeit, ein Hautpflegemittel herzustellen, bei dem das wertvolle Coenzym $Q_{10}$ von der Hautzelle tatsächlich aufgenommen wird. Es wurde gezeigt, welche Probleme die Aufnahme fettlöslicher Substanzen im Verdauungstrakt aufgrund des "Umwegs" über die Micellenbildung aufwirft. Diese Problematik ist bei der Aufnahme fettlöslicher Substanzen über die Haut noch weitaus größer, weil dort eine körpereigene Micellenbildung fehlt, die eine messbare Aufnahme z.B. von Coenzym $Q_{10}$ überhaupt erst ermöglichen würde. Deshalb bietet wasserlöslichen Coenzym $Q_{10}$ für den kosmetischen Bereich unvergleichlich bessere Einsatzmöglichkeiten.

b) Der Getränkeindustrie bietet sich der große technische Vorteil, dass sich das wasserlösliche Coenzym $Q_{10}$ bestens zur Herstellung klarer Getränke mit hoher Attraktivität und enormem Zusatznutzen eignet (USA Patentschrift 6 048 566).

Das in dieser Schrift beschriebene Konzentrat ist jedoch für die Supplementierung in Kapseln oder anderen Darreichungsformen aufgrund seines hohen Wassergehaltes weniger geeignet.

In der japanischen Patentanmeldung Nr. 55 081813 ist eine Zusammensetzung beschrieben, die Coenzym $Q_{10}$, ein natürliches Öl und eine oberflächenaktive Substanz enthält. Dabei soll der Gehalt an natürlichem Öl mehr als das Fünffache des Coenzym $Q_{10}$-Gehalts und der Gehalt an der oberflächenaktiven Substanz das 0.01-fache bis zum 2-fachen des Coenzym $Q_{10}$-Gehalts betragen.

Aus dem Dokument US 6,300,377 B1 ist eine oral applizierbare, flüssige Zusammensetzung bekannt, die Ubichinon, 0,1 Gew% bis 50 Gew% eines oberflächenaktiven Mittels, einen Glycerinester in einer Menge von 0,1 Gew% bis 60 Gew% und ein Triglycerid mit 0,1 Gew% bis 25 Gew% der Zusammensetzung bekannt. Der Gehalt an Q10 kann bis zu 15 Gew% betragen und als oberflächenaktives Mittel wird unter anderem Polysorbat 80 empfohlen. Diese Zusammensetzung ist insofern aufwendig, als sie eine Mischung von vier Inhaltsstoffen erfordert.

In dem Dokument EP 0 617 957 A1 werden pharmzeutische Formulierungen in Kapselform beschrieben, bei denen Ubichinon in einer Mischung aus Isopropylmyristat oder Isopropylpalmitat mit einem speziellen Rizinusöl aufgelöst ist. Als Nahrungsergänzungsmittel eignen sich diese Formulierungen schon wegen des Rizinusölgehalts nicht.

Das Dokument EP 0179583 bezieht sich auf eine wasserfreie Zusammensetzung aus einem hydrophoben Wirkstoff und einem Emulgator, der Polysorbat 80 sein kann. Mit einem Ubichinon beschäftigt sich diese Schrift nicht.

Der Erfindung liegen deshalb Sinn und Zweck zugrunde, ein hochkonzentriertes, z.B. dreiprozentiges wasserfreies Coenzym $Q_{10}$-Konzentrat zu entwickeln, das bei Raum- oder Körpertemperatur (ohne zusätzliche Erwärmung) transparent und wasserlöslich ist und sich deshalb für Kapseln oder vergleichbare Darreichungsformen und für Kosmetika usw. besser technologisch verarbeitbar und besser bioverfügbar ist

Dazu sieht die Erfindung ein wasserlösliches, im Wesentlichen wasserfreies Ubichinon-Konzentrat vor, welches aus 30 Gewichtseinheiten Coenzym $Q_{10}$, 820 Gewichtseinheiten eines Emulgators mit einem HLB-Wert zwischen 9 und 16, zweckmäßig Polysorbat 80, sowie 150 Gewichtseinheiten Distelöl oder vergleichbares Pflanzenöl besteht.

Gemäß einer alternativen Lösung der Erfindungsaufgabe besteht ein wasserlösliches, im Wesentlichen wasserfreies Ubichinon-Konzentrat aus 30 Gewichtseinheiten Coenzym $Q_{10}$, 730 Gewichtseinheiten Emulgator mit einem HLB-Wert zwischen 9 und 16, zweckmäßig Polysorbat 80, 140 Gewichtseinheiten Distelöl oder vergleichbares Pflanzenöl und 100 Gewichtseinheiten Glycerin. Als weitere Alternative sieht die Erfindung ein höher dosiertes Ubichinon-Konzentrat vor, welches wasserlöslich und im Wesentlichen wasserfrei ist und aus 50 Gewichtseinheiten Coenzym $Q_{10}$, 790 Gewichtseinheiten Polysorbat 80 und 160 Gewichtseinheiten pflanzlichem Distelöl besteht.

Jedes der vorstehend genannten Konzentrate kann entweder in einer oral zu applizierenden Kapsel mit einer insbesondere gelatinefreien Hülle, oder als Zusatz zu einem Hautpflegemittel oder als Zusatz zu einem Zahnpflegemittel verwendet werden.

Ein oben erläutertes Konzentrat als erste Lösung der gestellten Aufgabe kann in der Weise hergestellt werden, dass 820 Gewichtseinheiten eines Emulgators mit einem HLB-Wert zwischen 9 und 16, zweckmäßig Polysorbat 80, auf 85 °C erhitzt, dann 30 Gewichtseinheiten Coenzym $Q_{10}$ hinzu gegeben und die Mischung unter Beibehaltung der Temperatur so lange gerührt wird, bis sie homogen und transparent geworden ist, anschließend dieser Mischung 150 Gewichtseinheiten Distelöl oder vergleichbares Pflanzenöl zugegeben werden, nachdem dieses zuvor ebenfalls auf 85 °C erwärmt wurde, und unter Beibehaltung der Temperatur von 85 °C so lange gerührt wird, bis die gesamte Mischung ebenfalls homogen geworden ist.

Ein Konzentrat nach der ersten Alternative kann so hergestellt werden, dass 730 Gewichtseinheiten Emulgator mit einem HLB-Wert zwischen 9 und 16, zweckmäßig Polysorbat 80, auf 85 °C erhitzt, dann 30 Gewichtseinheiten Coenzym $Q_{10}$ hinzu gegeben und die Mischung unter Beibehaltung der Temperatur so lange gerührt wird, bis sie homogen und transparent geworden ist, anschließend dieser Mischung 140 Gewichtseinheiten Distelöl und 100 Gewichtseinheiten Glycerin zugegeben werden, nachdem diese zuvor ebenfalls auf 85 °C erwärmt wurden, und unter Beibehaltung der Temperatur von 85 °C so lange gerührt wird, bis die gesamte Mischung homogen und transparent geworden ist.

Zur Herstellung der zweiten Alternative geht man so vor: 790 Gewichtseinheiten Polysorbat 80 werden auf 85°C erhitzt, dann 50 Gewichtseinheiten Coenzym $Q_{10}$ hinzu gibt und die Mischung unter Beibehaltung der Temperatur von 85 °C so lange rührt, bis sie homogen und transparent geworden ist, anschließend dieser Mischung 160 Gewichtseinheiten pflanzliches Distelöl zugibt, nachdem dieses transparent geworden ist, anschließend dieser Mischung 150 Gewichtseinheiten Distelöl oder vergleichbares Pflanzenöl zugegeben werden, nachdem dieses zuvor ebenfalls auf 85 °C erwärmt wurde, und unter Beibehaltung der Temperatur von 85 °C so lange gerührt wird, bis die gesamte Mischung ebenfalls homogen geworden ist.

Als leichtes Öl kommen vor allem pflanzliche Öle mit einem hohen Anteil an Triglyceriden wie etwa $\alpha$-Linolensäure, $\gamma$-Linolensäure, Linolsäure, Ölsäure in Betracht. So enthält beipielsweise Distelöl bis zu 83% Linolsäure und bis zu 24% Ölsäure. Leinöl, das ebenfalls für den erfindungsgemäßen Zweck eingesetzt werden kann, enthält bis zu 71% Linolensäure, bis zu 31% Linolsäure und bis zu 23% Ölsäure. Sonnenblumenöl, Sojaöl und Olivenöl enthalten entsprechende Bestandteile, so daß auch diese Öle für die Erfindung als leichte Öle verwendet werden können. Weitere zweckmäßige Merkmale des erfindungsgemäßen Konzentrats sind in den Unteransprüchen angegeben.

Neben der Möglichkeit der direkten Anwendung des neuen Produkts in Kapseln oder vergleichbaren Darreichungsformen und in Kosmetika usw. bietet das neue Produkt im Vergleich zu wäßrigen Varianten die Vorteile, daß aufgrund seiner zähflüssigen Eigenschaft die Reaktions- und somit die Abbaugeschwindigkeit im Produkt herabgesetzt werden und daß bei längerer Lagerung keine Sedimente bzw. Bodensatz gebildet werden. Durch den Zusatz der genannten Öle wird eine mögliche Kristallisation im Konzentrat bei Körpertemperatur wirksam verhindert, so daß die Erfindung eine wesentliche Verbesserung gegenüber der US Patentschrift 6,048,566 darstellt Das in dieser patentschrift beschriebene Grundkonzentrat wäre unverdünnt bzw, wasserfrei erst bei über 45°C, d.h. bei weit höherer Temperatur als der Körpertemperatur (37°C) wasserlöslich.

Zur Erläuterung der Erfindung werden nachfolgend einige Herstellungsbeispiele für das Konzentrat mitgeteilt:

**Herstellungsbeispiel 1:**

Material: 1.) 30 g reines Coenzym $Q_{10}$ (gelbes Pulver)

2.) 820 g Emulgator mit einem HLB-Wert zwischen 9 und 16, vorzugsweise Polyoxyethylen-Sorbitanmonooleat (Polysorbat 80, Lamesorb SMO 20)

3.) 150 g Distelöl oder vergleichbares Pflanzenöl

Methode: 820 g Emulgator mit einem HLB-Wert zwischen 9 und 16, vorzugsweise Polysorbat 80, werden auf ca. 85°C erhitzt. Dann werden 30 g reines Coenzym $Q_{10}$ (gelbes Pulver) hinzugegeben und die Mischung (Gesamtmenge 850 g) unter Beibehaltung der Temperatur von ca. 85°C so lange (ca. 5 Minuten) gerührt, bis sie homogen und transparent geworden ist. Anschließend werden dieser Mischung 150 g Distelöl oder vergleichbares Pflanzenöl zugegeben, nachdem dieses zuvor ebenfalls auf ca. 85°C erwärmt wurde und unter Beibehaltung der Temperatur von ca. 85°C so lange (ca. 2 Minuten) gerührt, bis die gesamte Mischung (1.000 g) ebenfalls homogen und transparent geworden ist. Nach Abkühlung auf Raum- oder Körpertemperatur bleiben Klarheit und Wasserlöslichkeit erhalten.

Trübung: Die Messung der Trübung erfolgte mit dem Turb 550 bzw. Turb 550 IR der Firma WTW und folgt den Empfehlungen der US EPA bzw. entspricht der ISO 7027 / DIN 27027. Die Trübungsmessung einer 0,01-prozentigen Verdünnung der vorstehenden Mischung mit Wasser, was 100 mg Coenzym $Q_{10}$ pro Liter und somit dem dreifachen Tagesbedarf entspricht, ergab den Meßwert

$$3,0 \pm 0,2 \text{ bei Raumtemperatur}$$

auf der Skala von 1 bis 1.000. Bei Werten zwischen 1,0 und 10,0 gilt die gemessene Substanz als klar.

**Herstellungsbeispiel 2:**

Material: 1.) 50 g reines Coenzym Q 10 (gelbes Pulver)

2.) 790 g Emulgator Polysorbat 80

3.) 160 g pflanzliches Distelöl

Methode: 790 g Polysorbat 80 werden auf ca. 85°C erhitzt. Dann werden 50 g Coenzym Q 10 hinzugegeben und die Mischung (840 g) unter Beibehaltung der Temepratur von ca. 85°C so lange (etwa 5 Minuten) gerührt, bis sie homogen und transparent geworden ist. Anschließend werden dieser Mischung 160 g Distelöl zugegeben, nach dem dieses zuvor ebenfalls auf ca. 85°C erwärmt worden war, und unter Beibehaltung der Temperatur von ca. 85°C so lange (etwa 2 Minuten) gerührt, bis die gesamte Mischung (1000 g) ebenfalls homogen und transparent geworden ist. Nach Abkühlung auf Raum- oder Körpertemperatur bleiben Klarheit und Wasserlöslichkeit erhalten.

Trübung: Die Trübungsmessung wurde auf die im Beispiel 1 genannte Weise ausgeführt, wobei wieder eine 0,01prozentige Verdünnung der vorstehenden Mischung mit Wasser benutzt wurde. Es ergab sich ein Meßwert von

$$5,0 +/- 0,2 \text{ bei Raumtemperatu}$$

auf der Skala von 1 bis 1000, so daß Klarheit vorliegt.

**Herstellungsbeispiel 3:**

Material: 1.) 30 g reines Coenzym $Q_{10}$ (gelbes Pulver)
2.) 730 g Emulgator mit einem HLB-Wert zwischen 9 und 16, vorzugsweise Polyoxyethylen-Sorbitanmonooleat (Polysorbat 80, Lamesorb SMO 20)
3.) 140 g Distelöl oder vergleichbares Pflanzenöl
4.) 100 g Glycerin

Methode:     730 g Emulgator mit einem HLB-Wert zwischen 9 und 16, vorzugsweise Polysorbat 80, werden auf ca. 85°C erhitzt. Dann werden 30 g reines Coenzym $Q_{10}$ (gelbes Pulver) hinzugegeben und die Mischung (Gesamtmenge 760 g) unter Beibehaltung der Temperatur von ca. 85°C so lange (ca. 5 Minuten) gerührt, bis sie homogen und transparent geworden ist. Anschließend werden dieser Mischung 140 g Distelöl und 100 g Glycerin zugegeben, nachdem diese zuvor ebenfalls auf ca. 85°C erwärmt wurden und unter Beibehaltung der Temperatur von ca. 85°C so lange (ca. 2 Minuten) gerührt, bis die gesamte Mischung (1.000 g) ebenfalls homogen und transparent geworden ist.

Trübung:     Die Messung der Trübung erfolgte mit dem Turb 550 bzw. Turb 550 IR der Firma WTW und folgt den Empfehlungen der US EPA bzw. entspricht der ISO 7027 / DIN 27027. Die Trübungsmessung einer 0,01-prozentigen Verdünnung der vorstehenden Mischung mit Wasser, was 100 mg Coenzym $Q_{10}$ pro Liter und somit dem dreifachen Tagesbedarf entspricht, ergab den Meßwert

$$4,0 \pm 0,2 \text{ bei Raumtemperatur}$$

auf der Skala von 1 bis 1.000. Bei Werten zwischen 1,0 und 10,0 gilt die gemessene Substanz als klar.

Für eine geringere Q 10 Konzentration wie im vorstehenden Herstellungsbeispiel 3 genügt eine entsprechend geringere Emulgatorkonzentration. Das beigegebene wasserfreie Glycerin dient daher als Füllstoff zur Auffüllung der Mischung auf 1000 g. Selbstverständlich können auch andere wasserfreie Füllstoffe verwendet werden.

Die Empfehlung zur Herstellung von fünfprozentigem wasserlöslichen Coenzym $Q_{10}$-Konzentraten wird wie folgt begründet:

1) eine höhere Coenzym $Q_{10}$-Konzentration als fünf Prozent birgt das Risiko einer Auskristallisation des enthaltenen Coenzym $Q_{10}$, was die Wasserlöslichkeit des Konzentrats gefährden bzw. herabsetzen könnte.

2) Eine niedrigere Coenzym $Q_{10}$-Konzentration als drei Prozent hätte zur Folge, daß zur Deckung des Tagesbedarfs eine größere Konzentratmenge mit einem entsprechend höheren Volumen erforderlich wäre, das allerdings für eine Verarbeitung in Kapseln zu groß wäre.

Ein Gramm des dreiprozentigen wasserlöslichen Coenzym $Q_{10}$-Konzentrats enthält 30 mg Coenzym $Q_{10}$. Diese Menge entspricht dem empfohlenen Tagesbedarf und läßt sich vom Volumen her in Kapseln verarbeiten.

Ausgehend von der weitaus besseren Bioverfügbarkeit des wasserlöslichen Coenzym $Q_{10}$-Konzentrats ist zur Deckung des bisher allgemein empfohlenen Tagesbedarfs von 30 mg Coenzym $Q_{10}$ nur noch maximal die Hälfte - d.h. 15 mg Coenzym $Q_{10}$ in wasserlöslicher Form (erfindungsgemäßer Variante) - erforderlich bzw. empfehlenswert. Diese Coenzym $Q_{10}$-Menge (15 mg) ist in 500 mg des wasserlöslichen Konzentrats enthalten.

Eine Kapsel, die 500 mg wasserlösliches Coenzym $Q_{10}$-Konzentrat nach Herstellungsbeispiel 3 mit 15 mg Coenzym $Q_{10}$ enthält, enthält eine geringere Menge des Emulgators Polyoxyethylen-Sorbitanmonooleat (Polysorbat 80, Lamesorb SMO 20), die im Rahmen des Lebensmittelrechts täglich maximal aufgenommen werden darf. Produkttechnologisch sind dem Zusatz des Emulgators Polyoxyethylen-Sorbitanmonooleat keine Mengengrenzen gesetzt. Dabei gilt die Regelung "quantum satis".

Die nach den vorstehenden Herstellungsbeispielen 1 bis 3 entstandenen Konzentrate werden nach Abkühlung auf Raumtemperatur cremig und undurchsichtig. Bei Erwärmung auf Körpertemperatur werden die Konzentrate transparent, (zäh-) flüssig und mit temperiertem Wasser (ca. 37°C) leicht mischbar. Die Mischung dieser Konzentrate mit klarem Wasser ergibt eine vollkommen klare, stabile und magensäureresistente Lösung, in der sich das darin enthaltene Coenzym $Q_{10}$ in Form von micellenartigen Einheiten befindet, die

a) im Dünndarmbereich wie wasserlösliche Substanzen ohne Beteiligung von Gallensalzen vierfach schneller und quantitativ besser aufgenommen werden, und

b) es erstmalig ermöglichen, daß eine ursprünglich fettlösliche Substanz in größerer Menge (im kosmetischen Bereich) in die tiefen Hautschichten eindringt.

Neben Kapseln lassen sich die nach den vorstehenden Herstellungsbeispielen 1 bis 3 entstandenen Konzentrate bei weicher und/oder harter Gelierung als Laminat und/oder Füllung in verschiedene Lebensmittel wie Schokolade usw. und Kaugummis verarbeiten. In unverdünnter, vorzugsweise jedoch verdünnter Form können die vorstehend genannten Konzentrate in Tropfflaschen oder Trinkampullen verpackt werden. Darüber hinaus lassen sich die Konzentrate in Zahnpflege- und -reinigungsmittel (Zahncremes) einbringen.

**Patentansprüche**

1. Wasserlösliches, im Wesentlichen wasserfreies Ubichinon-Konzentrat, bestehend aus 30 Gewichtseinheiten Coenzym $Q_{10}$, 820 Gewichtseinheiten eines Emulgators mit einem HLB-Wert zwischen 9 und 16 sowie 150 Gewichtseinheiten Distelöl oder vergleichbares Pflanzenöl.

2. Wasserlösliches, im Wesentlichen wasserfreies Ubichinon-Konzentrat, bestehend aus 30 Gewichtseinheiten Coenzym $Q_{10}$, 730 Gewichtseinheiten Emulgator mit einem HLB-Wert zwischen 9 und 16, 140 Gewichtseinheiten Distelöl oder vergleichbares Pflanzenöl sowie 100 Gewichtseinheiten Glycerin.

3. Konzentrat nach Anspruch 1 oder 2, bei dem der Emulgator Polysorbat 80 ist.

4. Wasserlösliches, im Wesentliches wasserfreies Ubichinon-Konzentrat, bestehend aus 50 Gewichtseinheiten Coenzym $Q_{10}$, 790 Gewichtseinheiten Polysorbat 80 und 160 Gewichtseinheiten pflanzliches Distelöl.

5. Oral zu applizierende Kapsel mit einer insbesondere gelatinefreien Hülle, welche ein Konzentrat nach einem oder mehreren der vorherigen Ansprüche enthält.

6. Hautpflegemittel mit einem Zusatz eines Konzentrats nach einem oder mehreren der Ansprüche 1 bis 4.

7. Zahnpflegemittel mit einem Zusatz eines Konzentrats nach einem oder mehreren der Ansprüche 1 bis 4.

8. Verfahren zur Herstellung eines Konzentrats nach Anspruch 1 oder 3, bei dem 820 Gewichtseinheiten eines Emulgators mit einem HLB-Wert zwischen 9 und 16 auf 85°C erhitzt, dann 30 Gewichtseinheiten Coenzym $Q_{10}$ hinzugegeben und die Mischung unter Beibehaltung der Temperatur so lange gerührt wird, bis sie homogen und transparent geworden ist, anschließend dieser Mischung 150 Gewichtseinheiten Distelöl oder vergleichbares Pflanzenöl zugegeben werden, nachdem dieses zuvor ebenfalls auf 85°C erwärmt wurde, und unter Beibehaltung der Temperatur von 85°C so lange gerührt wird, bis die gesamte Mischung ebenfalls homogen und transparent geworden ist.

9. Verfahren zur Herstellung eines Konzentrats nach Anspruch 2 oder 3, bei dem 730 Gewichtseinheiten Emulgator mit einem HLB-Wert zwischen 9 und 16 auf 85°C erhitzt, dann 30 Gewichtseinheiten Coenzym $Q_{10}$ hinzugegeben und die Mischung unter Beibehaltung der Temperatur so lange gerührt wird, bis sie homogen und transparent geworden ist, anschließend dieser Mischung 140 Gewichtseinheiten Distelöl und 100 Gewichtseinheiten Glycerin zugegeben werden, nachdem diese zuvor ebenfalls auf 85°C erwärmt wurden, und unter Beibehaltung der Temperatur von 85°C so lange gerührt wird, bis die gesamte Mischung homogen und transparent geworden ist.

10. Verfahren zur Herstellung eines Konzentrats nach Anspruch 4, bei dem 790 Gewichtseinheiten Polysorbat 80 auf 85°C erhitzt, dann 50 Gewichtseinheiten Coenzym $Q_{10}$ hinzugegeben und die Mischung unter Beibehaltung der Temperatur von 85°C so lange gerührt wird, bis sie homogen und transparent geworden ist, anschließend dieser Mischung 160 Gewichtseinheiten Distelöl zugegeben werden, nachdem dieses zuvor auf 85°C erwärmt worden ist, und unter Beibehaltung der Temperatur von 85°C so lange gerührt wird, bis die gesamte Mischung ebenfalls homogen und transparent geworden ist.

11. Verfahren nach Anspruch 8 oder 9, **dadurch gekennzeichnet, dass** als Emulgator Polysorbat 80 eingesetzt wird.

**Claims**

1. Water-soluble, substantially anhydrous ubichinon concentrate consisting of 30 weight units of coenzyme $Q_{10}$, 820 weight units of an emulsifier having an HLB value between 9 and 16, and 150 weight units of safflower oil or a comparable vegetable oil.

2. Water-soluble, substantially anhydrous ubichinon concentrate consisting of 30 weight units of coenzyme $Q_{10}$, 730 weight units of an emulsifier having an HLB value between 9 and 16, 140 weight units of safflower oil or a comparable vegetable oil, and 100 weight units of glycerol.

3. Concentrate as claimed in Claim 1 or 2, wherein the emulsifier is polysorbate 80.

4. Water-soluble, substantially anhydrous ubichinon concentrate consisting of 50 weight units of coenzyme $Q_{10}$, 790 weight units of polysorbate 80 and 160 weight units of vegetable safflower oil.

5. Capsule to be applied orally, having a particularly gelatin-free shell and containing a concentrate as claimed in any one or several of the preceding Claims.

**6.** Skin care product with the addition of a concentrate as claimed in any one or several of Claims 1 to 4.

**7.** Dental care product with the addition of a concentrate as claimed in any one or several of Claims 1 to 4.

**8.** Method of producing a concentrate as claimed in Claim 1 or 3, wherein 820 weight units of an emulsifier having an HLB value between 9 and 16 are heated to 85°C, then 30 weight units of coenzyme $Q_{10}$ are added and the mixture is stirred, whilst maintaining the temperature, until it has become homogenous and transparent, then 150 weight units of safflower oil or a comparable vegetable oil are added to this mixture after having been heated likewise to 85°C beforehand, and the entire mixture is stirred, whilst maintaining the temperature of 85°C, until it has also become homogenous arid transparent.

**9.** Method of producing a concentrate as claimed in Claim 2 or 3, wherein 730 weight units of an emulsifier having an HLB value between 9 and 16 are heated to 85°C, then 30 weight units of coenzyme $Q_{10}$ are added and the mixture is stirred, whilst maintaining the temperature, until it has become homogenous and transparent, then 140 weight units of safflower oil and 100 weight units of glycerol are added to this mixture after having been heated likewise to 85°C beforehand, and the entire mixture is stirred, whilst maintaining the temperature of 85°C, until it has become homogenous and transparent.

**10.** Method of producing a concentrate as claimed in Claim 4. wherein 790 weight units of polysorbate 80 are heated to 85°C, then 50 weight units of coenzyme $Q_{10}$ are added and the mixture is stirred, whilst maintaining the temperature of 85°C, until it has become homogenous and transparent, then 160 weight units of safflower oil are added to this mixture after having been heated to 85°C beforehand, and the entire mixture is stirred, whilst maintaining the temperature of 85°C, until it has also become homogenous and transparent.

**11.** Method as claimed in Claim 8 or 9, **characterised in that** polysorbate 80 is used as the emulsifier.

**Revendications**

**1.** Concentré hydrosoluble sensiblement anhydre d'ubiquinone, constitué de 30 unités en poids de coenzyme $Q_{10}$, de 820 unités en poids d'un émulsifiant d'équilibre hydrophile-lipophile compris entre 9 et 16 ainsi que de 250 unités en poids d'huile de chardon ou d'huile végétale similaire.

**2.** Concentré hydrosoluble sensiblement anhydre d'ubiquinone, constitué de 30 unités en poids de coenzyme $Q_{10}$, de 730 unités en poids d'émulsifiant d'équilibre hydrophile-lipophile compris entre 9 et 16, de 140 unités en poids d'huile de chardon ou d'huile végétale similaire ainsi que de 100 unités en poids de glycérol.

**3.** Concentré suivant la revendication 1 ou 2, dans lequel l'émulsifiant consiste en Polysorbate 80.

**4.** Concentré hydrosoluble sensiblement anhydre d'ubiquinone, constitué de 50 unités en poids de coenzyme $Q_{10}$, de 790 unités en poids de Polysorbate 80 et de 160 unités en poids d'huile végétale de chardon.

**5.** Gélule à administrer par voie orale comprenant une enveloppe en particulier dépourvue de gélatine, qui contient un concentré suivant une ou plusieurs des revendications précédentes.

**6.** Composition destinée aux soins de la peau, additionnée d'un concentré suivant une ou plusieurs des revendications 1 à 4.

**7.** Composition destinée aux soins dentaires, additionnée d'un concentré suivant une ou plusieurs des revendications 1 à 4.

**8.** Procédé de préparation d'un concentré suivant la revendication 1 ou 3, dans lequel 820 unités en poids d'un émulsifiant d'équilibre hydrophile-lipophile compris entre 9 et 16 sont chauffées à 85°C, puis 30 unités en poids de coenzyme $Q_{10}$ sont ajoutées et, la température étant entretenue, le mélange est agité jusqu'à ce qu'il soit devenu homogène et transparent, ensuite ce mélange est additionné de 150 unités en poids d'huile de chardon ou d'huile similaire après que cette huile a été préalablement chauffée également à 85°C et, la température de 85°C étant entretenue, le mélange est agité jusqu'à ce qu'il soit en totalité également devenu homogène et transparent.

**9.** Procédé de préparation d'un concentré suivant la revendication 2 ou 3, dans lequel 730 unités en poids d'un émulsifiant d'équilibre hydrophile-lipophile compris entre 9 et 16 sont chauffées à 85°C, puis 30 unités en poids de coenzyme $Q_{10}$ sont ajoutées et, la température étant entretenue, le mélange est agité jusqu'à ce qu'il soit devenu homogène et transparent, ensuite ce mélange est additionné de 140 unités en poids d'huile de chardon et de 100 unités en poids de glycérol après qu'ils ont été préalablement chauffés également à 85°C et, la température de 85°C étant entretenue, le mélange est agité jusqu'à ce qu'il soit en totalité également devenu homogène et transparent.

**10.** Procédé de préparation d'un concentré suivant la revendication 4, dans lequel 790 unités en poids de Polysorbate 80 sont chauffées à 85°C, puis 50 unités en poids de coenzyme $Q_{10}$ sont ajoutées et, la température de 85°C étant entretenue, le mélange est agité jusqu'à ce qu'il soit devenu homogène et transparent, ensuite ce mélange est additionné de 160 unités en poids d'huile de chardon après que cette huile a été préalablement chauffée à 85°C et, la température de 85°C étant entretenue, le mélange est agité jusqu'à ce qu'il soit en totalité également devenu homogène et transparent.

**11.** Procédé suivant la revendication 8 ou 9, **caractérisé en ce que** du Polysorbate 80 est utilisé comme émulsifiant.

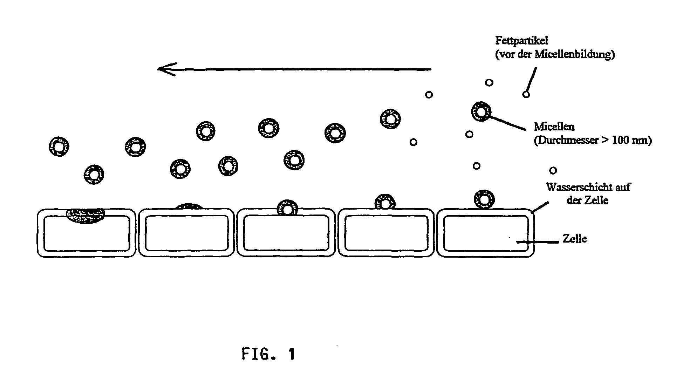

FIG. 1

Herstellungsmethode einer Emulsion
(Umkehrung durch Wärme und Säureeinflüsse)

| 1 | 2 | 3 |

unbehandelte Fettpartikel
in wäßriger Flüssigkeit

erschwerte Fettpartikel
mit E 444 oder E 445
in wäßriger Flüssigkeit

homogenisierte, erschwerte und stabilisierte
Fettpartikel mit E 444 oder E 445 und
E 414 oder E 1450 in wäßrigen Flüssigkeiten

Umkehrung, Phasentrennung (Fett/Wasser)
bei Erwärmung und nach Zugabe von Salzsäure
(Magensaft/Magensäure)

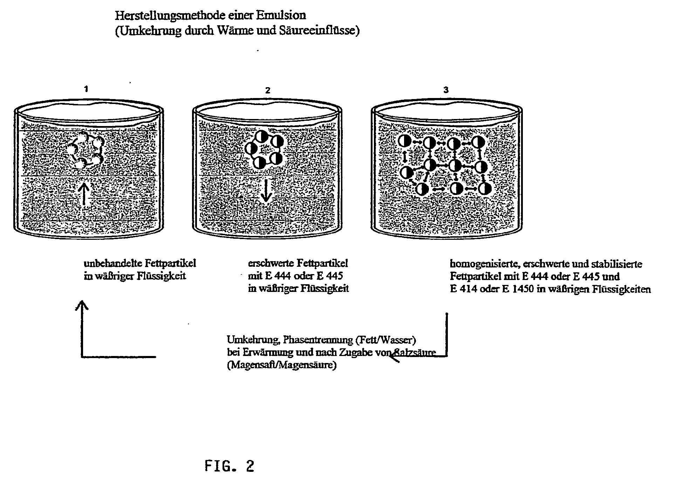

FIG. 2

Fettpartikel – Ø ~ 1000 nm

homogenes Fett-
Wasser-Gemisch
(trüb)

Die Verdauung erfolgt mit
Hilfe der Gallensalze

**Wasserlösliches Q$_{10}$**
**(Micellar-Mix)**

Micellen – Ø ~ 20 - 30 nm

Micellar-Mix
(klar)

Die Verdauung erfolgt ohne
Hilfe der Gallensalze

Micellar-Mix
(klar)

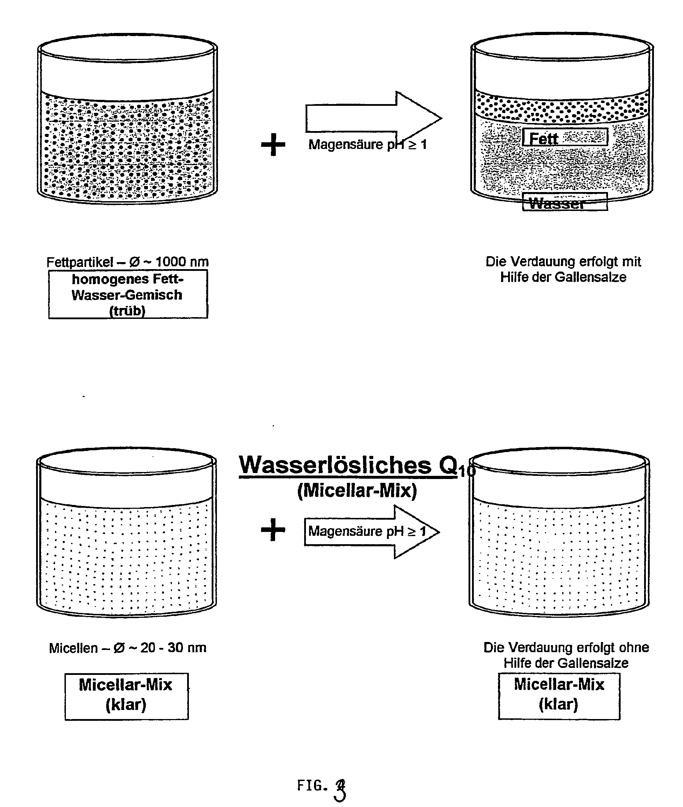

FIG. 3

**EP 1 404 291 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6048566 A
- JP 55081813 A
- US 6300377 B1
- EP 0617957 A1
- EP 0179583 A